# EUROPEAN PATENT SPECIFICATION

(11) **EP 3 134 147 B1**
(45) Date of publication and mention of the grant of the patent: **07.05.2025**
(21) Application number: 14882871.8
(22) Date of filing: 28.03.2014
(51) Int. Cl.: A61M 3/02, A61F 6/00

(54) **INTIMATE CLEANSER**
INTIMREINIGER
PRODUIT LAVANT À USAGE INTIME

(30) Priority: 21.02.2014 BR 202014004079 U
(43) Date of publication of application: 01.03.2017
(73) Proprietor: Mix For You Ltda., 11045-002 Santos SP (BR)
(72) Inventor: CASTROPIL LOGAARZO, Marcus Augustus, CEP 01155-901 São Paulo, SP (BR)
(74) Representative: Sarpi, Maurizio
(86) International application number: PCT/BR2014/000103
(87) International publication number: WO 2015/123742

(56) References cited:
- GB-A- 1 262 926
- US-A- 3 371 665
- US-A- 3 474 788
- US-A- 3 530 858
- US-A- 4 159 718
- US-A- 4 180 072
- US-A1- 2005 187 526
- US-A1- 2013 016 925
- US-B1- 6 190 366
- US-S- D 474 683

## Description

### TECHNICAL FIELD

The present invention relates to an intimate douche (500) comprising a bag (100), and a fixed and/or threadable applicator nozzle (200), in which the folding bag (100) presents a new practical and anatomic design, resulting in a portable and disposable intimate douche (500). Using the intimate douche, irrigaton of the pelvic cavity through the flow of content can be provided, preferably improved water expelled from the intimate douche (500) into the female and/or male pelvic cavity in order to assist in hygiene, cleansing, treating, odorizing, gut flora replacement, lubrication and/or anesthesia of such cavities.

### BACKGROUND OF THE INVENTION

The origin of the douches is a bit uncertain, although they exist for centuries. However, the douches have become popular in the United States in 1920, when Lysol and Zonite Products companies produced industrial quantities for commercial use.

Over time the intimate douches were commercially known for its use in the practice of contraceptive methods, intimate wash procedures and/or treatment of pelvic dysfunction.

Currently, there are only "female intimate vaginal douches" in the market in the form of pear or horn which are pouches and/or bulbs of flexible rubber connected to a nozzle, or even "enemas" comprised of a rubber bag and/or plastic bags attached to hoses for fluid instillation, and both devices result in large packages thereby inhibiting its discreet carriage by the users. However, these douches and/or enemas are mostly not comfortable and/or disposable, and much less hygienically consistent.

The female intimate douches are initially designed for vaginal use, in which when the woman uses it can see at least partially the area to be cleaned, being it only a simple wash and not stimulating the removal of urine waste, sperm or menstrual waste. However, the female intimate douches currently available in the market end up being used by the sodomite community to induce the stimulation of peristalsis, resulting in excretion of fecal waste as a hygienic procedure for the practice of anal sex, which is not an intended use for such device.

Particularly, considering the great concern for intimate hygiene in anal sex, especially between the practitioners of sodomy, there is an evident need to avoid the risk of undesirable residues, e.g., fecal residues in the intimate areas. However, over time, the sodomites have been using "adapted" equipment which are not practical and, especially, need very restricted environments for their application. It is also known that some individuals use PET bottles of water and/or soda adapted for such use, resulting in big anatomical problems and risks of injury during the cleaning procedure.

US 3,474,788 discloses a disposable douche that includes a plastic bag and a vaginal applicator having a front end expandable to pressure.

US 3,690,319 refers to a vaginal douche bag, and more particularly with a douche bag and a syringe of the disposable type for use in cleaning, disinfection and/or application of medication into the vagina.

US 4,159,718 provides a disposable douche made of two sheets of a thin plastic material sealed along their edges to form a bag with a closing end.

CN 2113726 relates to a utility model of a disposable sanitary washing device including a bag containing a fluid and a hose.

CN 2506189 displays a utility model of a partially portable washing device comprising a bag for water storage, a drain pipe, a jet pipe, a nozzle, a wall, a thermal plate for thawing, a box to receive the residue of thawing and a movement plug.

US 6,190,366 B1 discloses a portable washing device comprising a water container made from laminating a polyethylene or polypropylene type film with a synthetic resin film.

In order to overcome the known drawbacks of the prior art the Applicant developed a practical, anatomical, portable and disposable intimate douche for the hygiene of female and/or male pelvic cavities.

### DISCLOSURE OF THE INVENTION

The present invention relates to an intimate douche according to claim 1, the intimate douche (500) comprising a bag (100), and a fixed and/or threadable applicator nozzle (200), in which the folding bag (100) presents a new practical and anatomic design, resulting in a portable and disposable intimate douche (500). Additionally, the present disclosure provides irrigation of the pelvic cavity through the flow of content, preferably improved water expelled from the intimate douche (500) into the female and/or male pelvic cavity in order to assist in hygiene, cleansing, treating, odorizing, gut flora replacement, lubrication and/or anesthesia of such cavities.

### BRIEF DESCRIPTION OF THE DRAWINGS

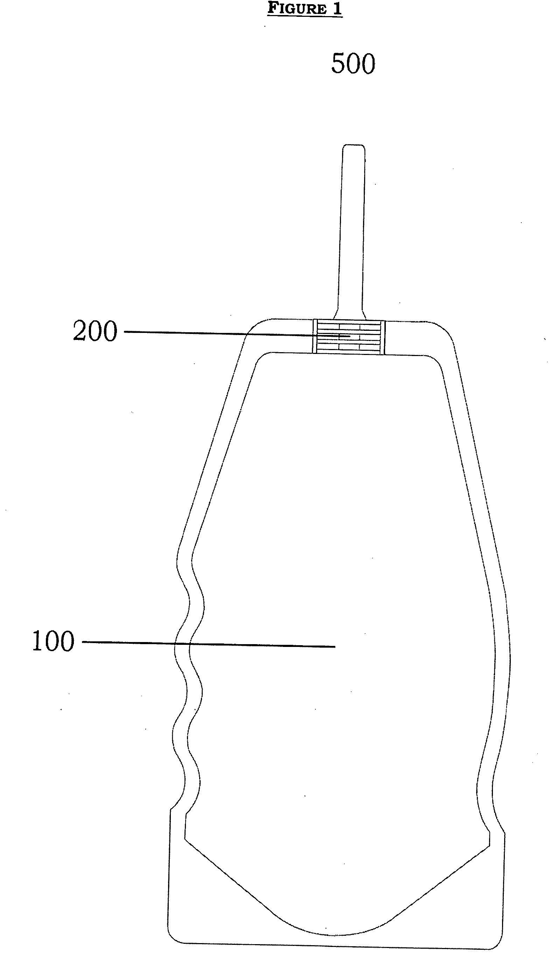

Figure 1 shows a plan front view of the intimate cleanser with fixed nozzle of the utility model;

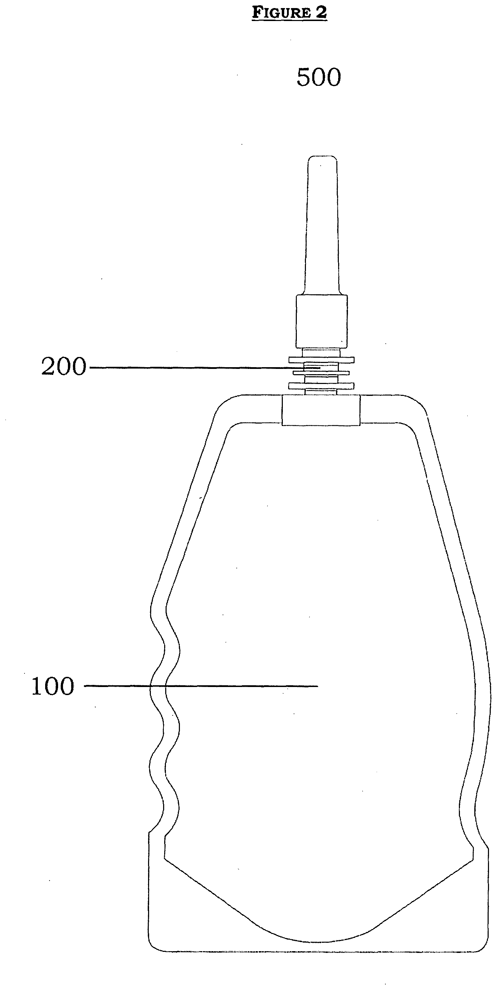

Figure 2 shows a plan front view of the intimate cleanser with removable nozzle of the utility model;

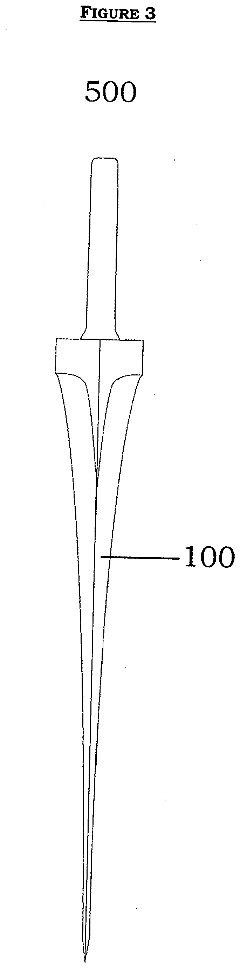

Figure 3 shows a side view of the empty intimate cleanser with a fixed nozzle;

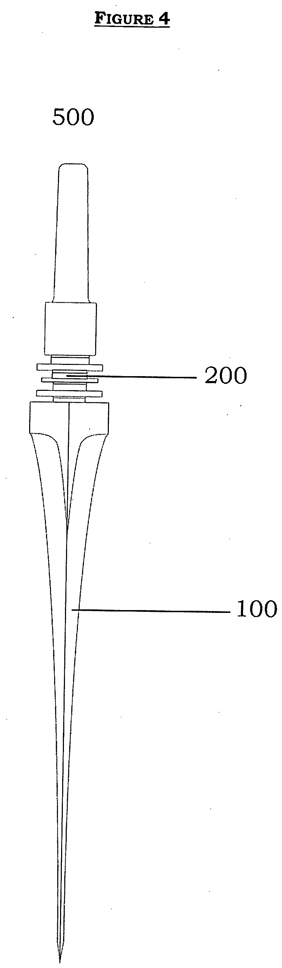

Figure 4 shows a side view of the empty intimate cleanser with a removable nozzle;

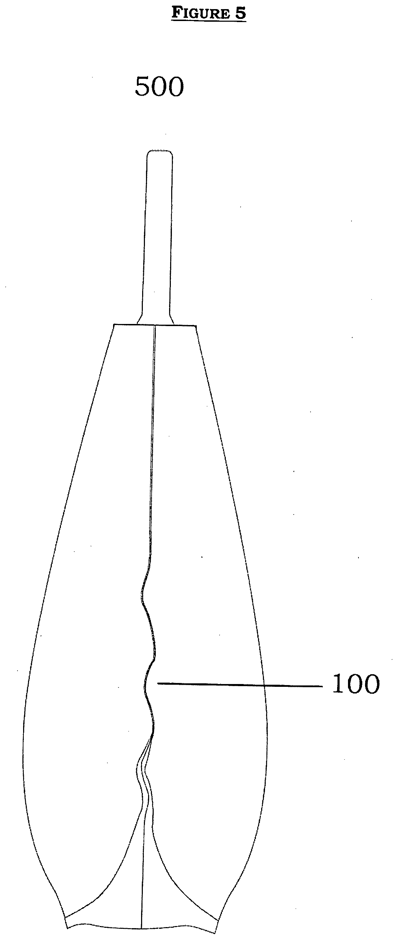

Figure 5 is a side view of the intimate cleanser with fixed nozzle in its state filled for use;

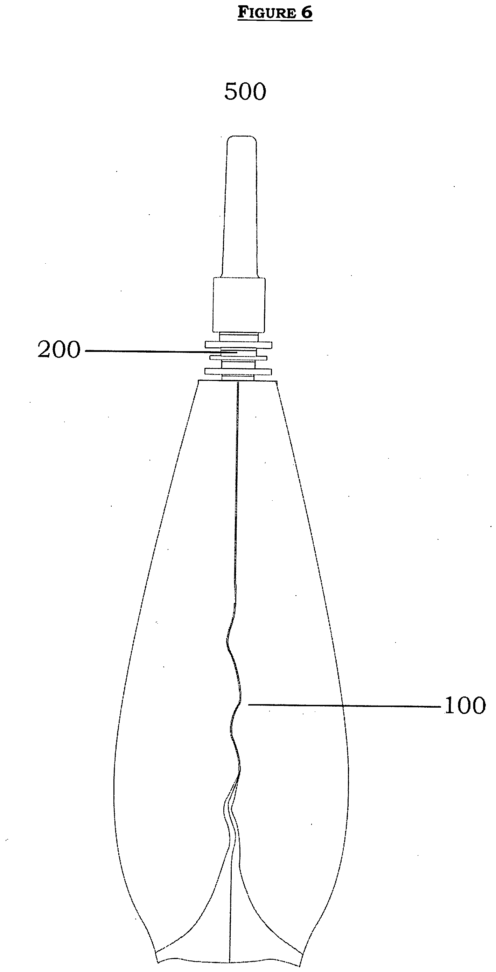

Figure 6 is a side view of the intimate cleanser with removable nozzle in its state filled for use;

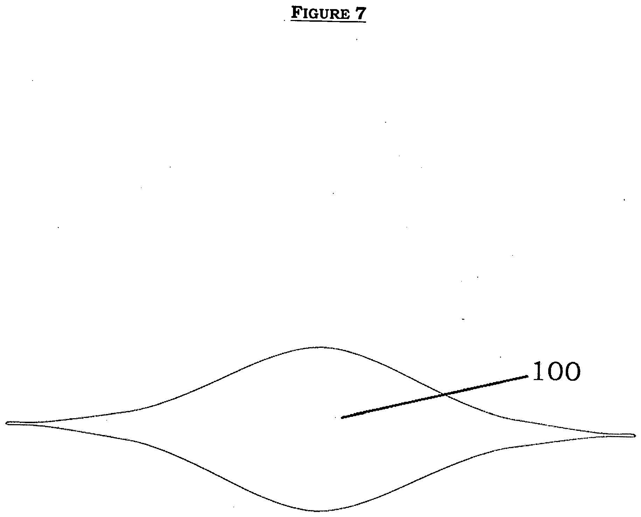

Figure 7 shows a bottom view of the utility model in its state filled for use;

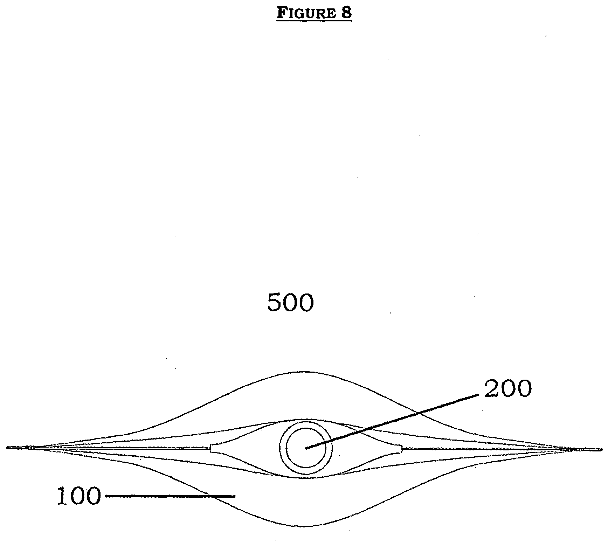

Figure 8 is a top view of the intimate cleanser with fixed nozzle in its state filled for use;

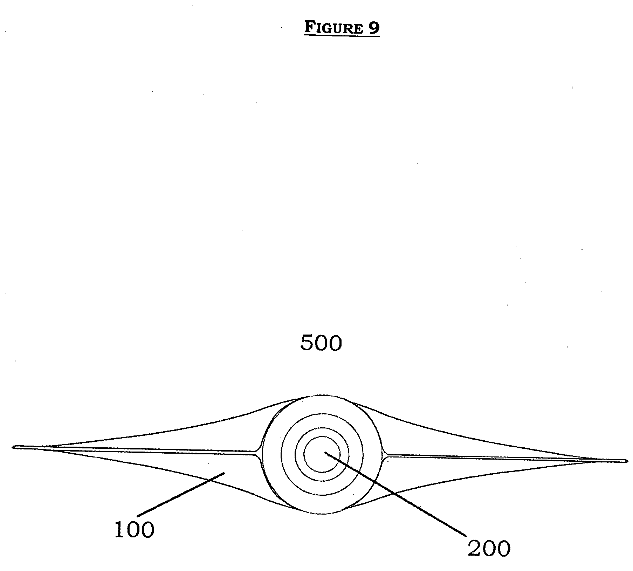

Figure 9 is a top view of the intimate cleanser with removable nozzle in its state filled for use;

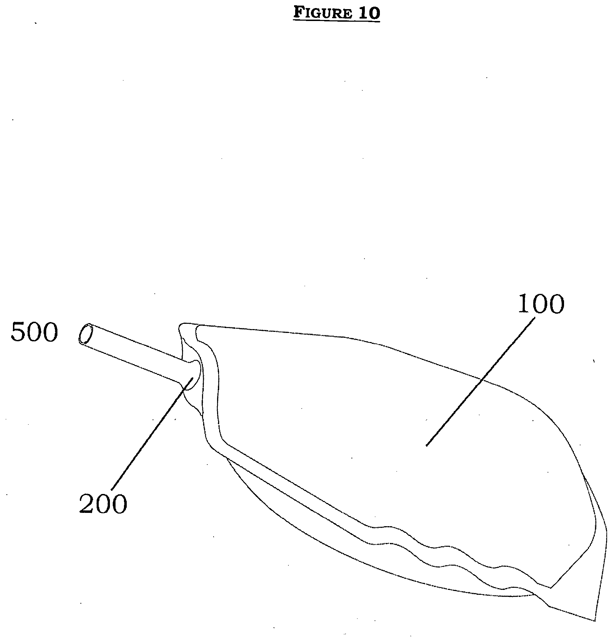

Figure 10 is a perspective view of the intimate cleanser with fixed nozzle in its state filled for use; and

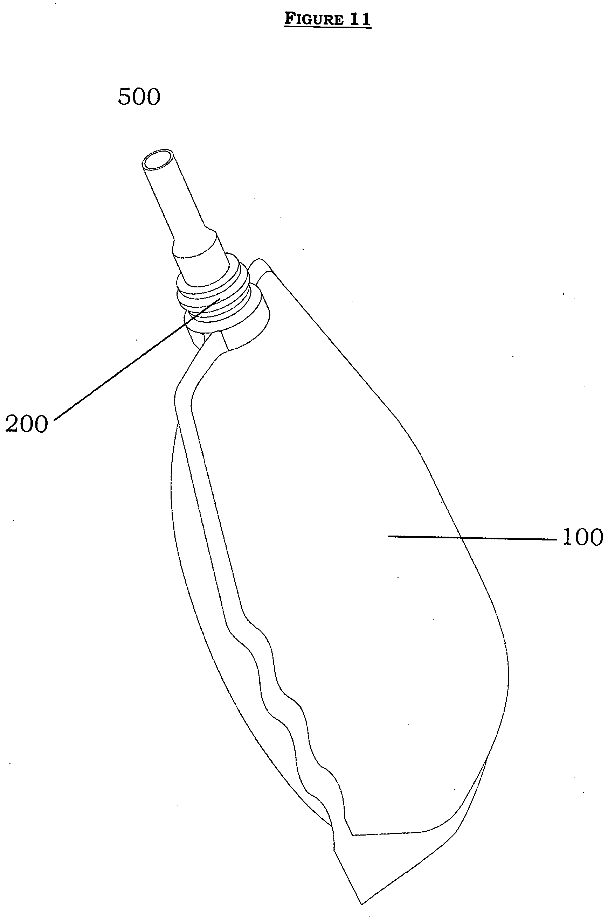

Figure 11 is a perspective view of the intimate cleanser with removable nozzle in its state filled for use.

### DETAILED DISCLOSURE OF THE INVENTION

In a first embodiment, the present invention refers to an intimate douche (500) having a bag (100) which comprises a multilayer polymeric film, preferably polyethylene terephthalate (PET) laminated with low density polyethylene and/or biaxially oriented *nylon* films, BOPP films, or PP, sealed along their ends through temperature melting and/or ultrasound to form the bag (100), without the need of reinforcing material at the junction of the bag (100) and the fixed and/or threadable applicator nozzle (200). Furthermore, the bag (100) is resistant to manual pressure exerted externally and contains an internal volume of 50 to 800 ml, preferably an internal volume of 300 ml, and foldable when empty for conditioning the intimate douche (500) in the compact packages, such as packages of condoms.

Still, the intimate douche (500) presents a fixed and/or threadable applicator nozzle (200) made by a high density polymeric material, particularly nontoxic polyethylene resin being the applicator nozzle

(200) optionally removable. Additionally, the size of the fixed and/or threadable applicator nozzle (200) may vary from 30 to 150 mm, preferably 60 mm.

In a second aspect, which is not part of the claimed invention, the present disclosure relates to the use of the intimate douche (500) which provides irrigation of the female and/or male pelvic cavities through the flow of contents such as liquid dosage forms, such as solutions, suspensions, cavity solutions as enema, eteroclism and/or clisters, and preferably improved water, semi-solid forms such as gels, and solid forms such as powders, granules, vaginal tablets, ovules, suppositories contained in the bag (100) from the intimate douche (500) into the female and/or male pelvic cavity in order to assist in hygiene, cleansing, treating, odorizing, gut flora replacement, lubrication and/or anesthesia of such cavities.

Additionally, the content may include solutes such as gut flora replenisher, flavoring, anesthetics and/or lubricants dissolved in solvents, preferably improved water.

### BEST MODE FOR CARRYING OUT THE INVENTION

The procedure for applying the intimate douche (500) is accomplished through the steps of (a) opening the seal of secondary package, (b) unfolding the bag (100), (c) filling the bag (100) with the content of interest, preferably pure improved water until the indication in the bag (100), (d) threading the threadable applicator nozzle (200), (e) introducing into the pelvic cavity of interest and (f) pressing the bag (100) so that the content of the bag is released into the cavity to be cleansed. Repeat the operation three times or until the liquid comes clear. After cleaning dispose all the items used.

### VAGINAL APPLICATION

Regarding the application of intimate douche (500) in the vaginal cavity, the washing procedure is intended for removing menstrual waste, semen or other secretions arising from infections. The intimate douche (500) can be used as an aid to the application of the medication indicated in the treatment of diseases in the vaginal region. Still, their use is recommended for preventive hygiene of the intimate area, bringing benefit to the prevention of diseases resulting from poor or lack of hygiene in the region.

For example, the procedures for the application of the intimate vaginal douche (500) comprises the steps of (a) with a shower fitted, filling or aspirating the improved water and/or the solution to be used with the tip of the douche, (b) inserting the applicator nozzle (200) into the vaginal cavity, (c) irrigating the vaginal cavity through manual pressure of the bag (100) and (d) after introducing the liquid into the cavity, retaining the bag (100) compressed during removal to prevent the solution from returning to the intimate douche (500). After use, the intimate douche (500) should be discarded, thus ensuring personal hygiene to those who used it.

### ANAL APPLICATION

As an example of use after the procedures of assembling, filling and applying of the intimate douche (500) into the anal cavity cleaning will happen from the distal portion of the large intestine and through the entire length of the rectum to the anal canal. Thus it is supposed that the cleaning procedure with the intimate douche (500) is carried out at least two or more times in order to result in an appropriate cleaning procedure. Still, the intimate douche (500) cleans the most proximal portion of the anal cavity and, in particular, provides the stimulation of peristalsis in the sigmoid (interconnection of the large intestine and the rectum) by water contact in the dentate / pectineal line so that the fecal waste is expelled from the distal region of the rectum by the washing of the anal canal. After use, the intimate douche (500) should be discarded, thus ensuring personal hygiene to those who used it.

## Claims

1. Intimate douche (500) for internal use, in particular for irrigation of a pelvic cavity, said intimate douche (500) comprising a bag (100) and a fixed and/or threadable applicator nozzle (200), wherein the bag (100) is foldable and presents a new practical and anatomic design, resulting in a portable and disposable intimate douche (500), **characterised in that** the bag (100) comprises a multilayer polymeric film made of polyethylene terephthalate (PET) laminated with low density polyethylene and/ or biaxially oriented nylon films, BOPP films, or PP, sealed along their ends.

2. Intimate douche (500) according to claim 1, **characterized in that** the bag (100) comprises an internal volume of 50 to 800 ml, preferably an internal volume of 300 ml.

3. Intimate douche (500) according to claim 1, wherein the bag (100) is foldable when empty for conditioning the intimate douche (500) in compact packages, such as packages of condoms.

4. Intimate douche (500) according to claim 1, wherein the fixed and/or threadable applicator nozzle (200) comprises a high density polymeric material, particularly nontoxic and optionally removable polyethylene resin.

5. Intimate douche (500) according to claim 1, wherein the fixed and/or threadable applicator nozzle (200) comprises sizes of 30 to 150 mm, preferably of 60 mm.

## Patentansprüche

1. Intim-Dusche (500) für den internen Gebrauch, insbesondere zur Spülung einer Beckenhöhle, wobei die Intim-Dusche (500) einen Beutel (100) und eine feste und/oder aufschraubbare Applikatordüse (200) umfasst, **dadurch gekennzeichnet, dass** der Beutel (100) faltbar ist und ein neues praktisches und anatomisches Design aufweist, wodurch eine tragbare und wegwerfbare Intim-Dusche (500) entsteht, **dadurch gekennzeichnet, dass** der Beutel (100) eine mehrschichtige Polymerfolie aus Polyethylenterephthalat (PET) umfasst, die mit Polyethylen niedriger Dichte und/oder biaxial orientierten Nylonfolien, BOPP-Folien oder PP laminiert ist und entlang ihrer Enden versiegelt ist.

2. Intim-Dusche (500) gemäß Anspruch 1, **dadurch gekennzeichnet, dass** der Beutel (100) ein internes Volumen von 50 bis 800 ml, vorzugsweise ein internes Volumen von 300 ml, umfasst.

3. Intim-Dusche (500) gemäß Anspruch 1, **dadurch gekennzeichnet, dass** der Beutel (100) faltbar ist, wenn er leer ist, um die Intim-Dusche (500) in kompakten Verpackungen, wie beispielsweise Kondomverpackungen, unterzubringen.

4. Intim-Dusche (500) gemäß Anspruch 1, **dadurch gekennzeichnet, dass** die feste und/oder aufschraubbare Applikatordüse (200) ein hochdichtes polymeres Material umfasst, insbesondere ungiftiges und optional abnehmbares Polyethylenharz.

5. Intim-Dusche (500) gemäß Anspruch 1, **dadurch gekennzeichnet, dass** die feste und/oder aufschraubbare Applikatordüse (200) Größen von 30 bis 150 mm, vorzugsweise 60 mm, umfasst.

## Revendications

1. Douche intime (500) à usage interne, notamment pour l'irrigation d'une cavité pelvienne, ladite douche intime (500) comprenant un sac (100) et un bec applicateur (200) fixe et/ou vissable, le sac (100) étant pliable et présentant une nouvelle conception pratique et anatomique, résultant en une douche intime (500) portable et jetable, **caractérisée en ce que** le sac (100) comprend un film polymère multicouche en polyéthylène téréphtalate (PET) laminé avec du polyéthylène basse densité et/ou des films de nylon biaxialement orientés, des films BOPP ou du PP et scellé le long de leurs extrémités.

2. Douche intime (500), selon la revendication 1, **caractérisée en ce que** le sac (100) comprend un volume interne de 50 à 800 ml, de préférence, un volume interne de 300 ml.

3. Douche intime (500), selon la revendication 1, **caractérisée en ce que** le sac (100) est pliable lorsqu'il est vide pour conditionner la douche intime (500) dans des emballages compacts, tels que des emballages de préservatifs.

4. Douche intime (500), selon la revendication 1, **caractérisée en ce que** le bec applicateur (200) fixe et/ou vissable comprend un matériau polymère de haute densité, notamment une résine de polyéthylène non toxique et éventuellement amovible.

5. Douche intime (500), selon la revendication 1, **caractérisée en ce que** le bec applicateur (200) fixe et/ou vissable comprend des dimensions de 30 à 150 mm, de préférence, de 60 mm.
